# EUROPÄISCHE PATENTSCHRIFT

(11) **EP 1 126 875 B1**
(45) Veröffentlichungstag und Bekanntmachung des Hinweises auf die Patenterteilung: **07.02.2007**
(21) Anmeldenummer: 99971718.4
(22) Anmeldetag: 08.11.1999
(51) Int. Cl.: A61K 39/145, A61K 39/295, A61K 9/127, A61K 39/39, B05B 11/00, A61P 11/02

(54) **ABGABEEINRICHTUNG FÜR EINE ZUSAMMENSETZUNG EINER PHARMAZEUTISCH WIRKSAMEN SUBSTANZ**
DISPENSING DEVICE FOR A PHARMACEUTICALLY ACTIVE COMPOSITION
DISPOSITIF DE DISTRIBUTION POUR UNE COMPOSITION D'UNE SUBSTANCE PHARMACEUTIQUEMENT ACTIVE

(30) Priorität: 06.11.1998 DE 19851282
(43) Veröffentlichungstag der Anmeldung: 29.08.2001
(73) Patentinhaber: Berna Biotech AG, 3018 Bern (CH)
(72) Erfinder: GLÜCK, Reinhard, CH-3095 Spiegel Bern (CH); GLÜCK, Ulrich, CH-6045 Meggen/Luzern (CH); COLLIOUD, André, CH-3073 Gümligen (CH)
(74) Vertreter: Vossius & Partner
(86) Internationale Anmeldenummer: PCT/EP1999/008557
(87) Internationale Veröffentlichungsnummer: WO 2000/027430

(56) Entgegenhaltungen:
- EP-A- 0 170 198
- EP-A- 0 412 524
- EP-A- 0 869 181
- FR-A- 1 257 220
- FR-A- 2 739 294
- FR-A- 2 764 807
- US-A- 2 434 875
- US-A- 5 182 109
- US-A- 5 797 390
- DATABASE MEDLINE [Online] US NATIONAL LIBRARY OF MEDICINE (NLM), BETHESDA, MD, US CRYZ S J JR ET AL: "Immunopotentiating reconstituted influenza virosomes as a novel antigen delivery system." retrieved from STN Database accession no. 1998214897 XP002140450 -& DEVELOPMENTS IN BIOLOGICAL STANDARDIZATION, (1998) 92 219-23., Bd. 92, 1998, Seiten 219-223, XP000914674
- FORD M J ET AL: "INTRANASAL IMMUNIZATION WITH INACTIVATED INFLUENZA VIRUS AND VIROSOMES" VACCINES (COLD SPRING HARBOR) 1991,1991, Seiten 209-216, XP000914630 ISSN: 0899-4072
- CHOUDHRY MA ET AL: "60-fold purification of cell-bound heat labile enterotoxin of Escherichia coli by simple affinity chromatography" MED SCI RES, Bd. 23, Nr. 4, 1995, Seiten 267-268, XP000920838
- BELSHE RB ET AL: "The efficacy of live attenuated, cold-adapted, trivalent, intranasal influenzavirus vaccine in children" N ENG J MED, Bd. 338, Nr. 20, Mai 1998 (1998-05), Seiten 1405-12, XP000914636
- MENGIARDI B ET AL: "Virosomes as carriers for combined vaccines" VACCINE, Bd. 13, Nr. 14, 1995, Seiten 1306-1315, XP002140447
- HAAN DE A ET AL: "MUCOSAL IMMUNOADJUVANT ACTIVITY OF LIPOSOMES: INDUCTION OF SYSTEMIC IGG AND SECRETORY IGA RESPONSES IN MICE BY INTRANASAL IMMUNIZATION WITH AN INFLUENZA SUBUNIT VACCINE AND COADMINISTERED LIPOSOMES" VACCINE, Bd. 13, Nr. 2, 1. Februar 1995 (1995-02-01), Seiten 155-162, XP000615909 ISSN: 0264-410X
- R I WALKER ET AL: "Use of Heat-labile Toxin of Enterotoxigenic Escherichia coli to Facilitate Mucosal Immunization" VACCINE RESEARCH,US,MARY ANN LIEBERT, INC., PUBLISHERS, Bd. 1, Nr. 2, 1. Januar 1993 (1993-01-01), Seiten 1-10, XP002077884
- PIERCE NF ET AL: "Procholeragenoid: A safe and effective antigen for oral immunization against experimental cholera" INFECTION AND IMMUNITY, Bd. 40, Nr. 3, 1983, Seiten 1112-11118, XP000914626 in der Anmeldung erwähnt
- GLÜCK R: "Adjuvant activity of immunopotentiating reconstituted influenza virosomes (IRIVs)" VACCINE, Bd. 17, März 1999 (1999-03), Seiten 1782-1787, XP002140448
- GLÜCK U ET AL: "Phase 1 evaluation of intranasal virosomal influenza vaccine with and without Escherichia coli Heat-Labile Toxin in adult volunteers" JOURNAL OF VIROLOGY, Bd. 73, Nr. 9, September 1999 (1999-09), Seiten 7780-7786, XP002140449

## Beschreibung

Die vorliegende Anmeldung betrifft Zusammensetrungen einer pharmazeutisch wirksamen Substanz, insbesondere Antigene, die mit einem mukosalen Adjuvans vermischt sind und mit einer als Sprayapplikator ausgebildeten Abgabeeinrichtung so auf die Nasenschleimhaut gebracht werden können, daß eine bisher noch nie erzielte Wirksamkeit erreicht werden kann.

Insbesondere beschreibt die Anmeldung eine Vakzine zur intranasalen Applikation, bestehend aus:
(a) Influenzaoberflächenproteinen, welche liposomal formuliert sind (Virosomen) ;
(b) mukosalem Adjuvans bakteriellen Ursprungs;
(c) spezifischem Sprayapplikator, der so konstruiert ist, daß nahezu 100% des Spraystosses vollumfänglich auf die für die Wirksamkeit wichtige Nasenschleimhaut appliziert werden kann.

Die Antigene sind vorzugsweise Influenza-Oberflächenglykoproteine, die liposomal formuliert sind (sog. Virosomen). Diese Virosomen werden mit einem mukosalen Adjuvans gemischt, das bakteriellen Ursprungs ist. Idealerweise kommt es aus der Klasse der aktiven oder inaktiven Toxine, wie *Heat Labile Toxin* (HLT), Choleratoxin (CT) oder Procholeragenoid (PCG). Der nasale Sprayapplikator ist so konstruiert, daß die wirksame Substanz im wesentlichen vollumfänglich auf die Nasenschleimhaut appliziert werden kann. Die der Erfindung zugrundeliegende Formel kann für verschiedene medizinische Indikationen verwendet werden, wie Impfung gegen Grippe oder andere Infektionskrankheiten sowie zur therapeutischen Behandlung von verstopfter Nase, Rekonstitution von lädierter Nasenschleimhaut oder allgemein mukosal lokalisierten Krankheiten.

Verschiedene Autoren haben die immunstimulierende Wirkung von Liposomen (künstlichen Membranen) beschrieben (Gregoriadis, G.: Immunological adjuvants: A role for liposomes. Immunol. Today 11 (1990) 89-97). Diese immunstimulierende Wirkung wird erzielt, wenn die Antigene auf die Oberfläche der Liposomen gekoppelt werden (Glück R, Mischler R, Finkel B, Que JU, Scarpa B, Cryz SJ Jr: Immunogenicity of new virosome influenza vaccine in the elderly people. Lancet 344 (1994) 160-163), im Innern der Liposomen eingeschlossen werden (Gregoriadis G, Davis D, Davis A: Liposomes as immunological adjuvants: Antigen incorporation studies. Vaccine 5 (1987) 145-151) oder nur mit Liposomen vermischt werden (De Haan A, Geerligs HJ, Huckshorn JP, Van Scharrenburg GJM, Palache AM, Wilschut J: Mucosal immunoadjuvant activity of liposomes: induction of systemic IgG and secretory IgA responses in mice by intranasal immunization with an influenza subunit vaccine and coadministered liposomes. Vaccine 13 (1995) 613-616). Eine zusätzliche immunstimulierende Wirkung wurde beobachtet, wenn solche Liposomen mit transmembranen, und, fusogenen Glykoproteinen "gespiked" wurden (z. B. Influenzaglykoproteine (Glück, R., Mischler, R., Brantschen, S., Just, M., Althaus, B., Cryz, S. J., Jr.: Immunopotentiating reconstituted influenza virosome (IRIV) vaccine delivery system for immunization against hepatitis A. J. Clin. Invest. 90 (1992) 2491-2495) oder Sendaiglykoproteine (Gould-Fogerite, S., Mazurkiewicz, J. E., Bhisitkul, D., Mannino, R. J.: The reconstitution of biologically active glycoproteins into large liposomes. Use as a delivery vehicle to animal cells. In: C. H. Kim, J. Diwan, H. Tedeschi und J. J. Salerno (Hrsg.), Advances in Membrane Biochemistry and Bioenergetics, Plenum Publishing Corp., New York (1987) S. 569).

Bisher wurde jedoch diese Wirkung meistens nach parenteraler Applikation (i.m., s.c. oder i.p.) beschrieben. Einige Autoren fanden jedoch, daß solche Formulierungen mit herkömmlichen Methoden (Tropfer oder herkömmliche Nasensprays) erfolgreich verabreicht werden können. Die Ergebnisse beziehen sich jedoch fast ausschließlich auf Experimente mit Labortieren, meist die Maus, welche im Vergleich zum Körpergewicht eine viel größere Nasenschleimhaut besitzt als vergleichsweise der Mensch. Zudem war die verabreichte Antigenmenge so hoch, daß alleine aus wirtschaftlichen sowie Gründen der Produktsicherheit eine Anwendung beim Menschen nicht in Frage käme. Auch konnte gezeigt werden, daß bei einer vernünftigen Dosierung des Antigens (Influenza) trotz liposomaler Formulierung und richtiger nasaler Applikation keine befriedigende Wirkung erzielt werden konnte.

Andere Autoren versuchten deshalb, eine wirksame nasal applizierbare Vakzine zu entwickeln, indem sie anstatt der liposomalen Formulierung die Antigene mit einem mukosalen Adjuvans bakteriellen Ursprungs vermischten. Dabei wurden insbesondere HLT, CT oder nicht toxische Derivate von HLT oder CT (Elson CO, Ealding W: Generalized systemic and mucosal immunity in mice after mucosal stimulation with cholera toxin. J. Immunol. 132 (1984) 2736-2741) verwendet.

Die Ergebnisse in der Literatur zeigten in der Tat vielversprechende. Wirkungen nach nasaler Applikation, welche erneut vor allem in Labortieren erzielt worden waren. Jedoch einige wenige klinische Versuche im Menschen bestätigten eine gewisse Wirksamkeit (Tamura S-J, Ishihira K, Miyata K, Aizawa C, Kurata T: Mechanism of enhancement of the immune responses to influenza vaccine with cholera toxin B subunit and a trace amount of hölotoxin. Vaccine 13 (1995) 339-341). Doch erneut war die zu verwendende Antigen- und Adjuvansmenge sehr hoch, was wiederum Vorbehalte betreffend Produktsicherheit und Möglichkeit der Kommerzialisierung offenließ.

Weitere Nachteile der bisher beschriebenen Versuche zur Entwicklung einer nasal applizierbaren, präventiv oder therapeutisch wirksamen Vakzine sind die heute zur Verfügung stehenden ungenügenden Applikatorsysteme.

Zur Zeit gibt es Nasensprays als Mono-, Bi- und Multidosis-Applikatoren. Eingehende Untersuchungen und Prüfungen der bisherigen Nasensprays mittels Farbstoffapplikation (Methylenblau) und Nasenendoskopie haben ergeben, daß die zu versprühende Substanz (Vakzine, pharmazeutische Lösung) nur höchstens zu 25% an die Schleimhaut des Mukosa-assoziierten Immunsystems der Turbinalien gelangt.

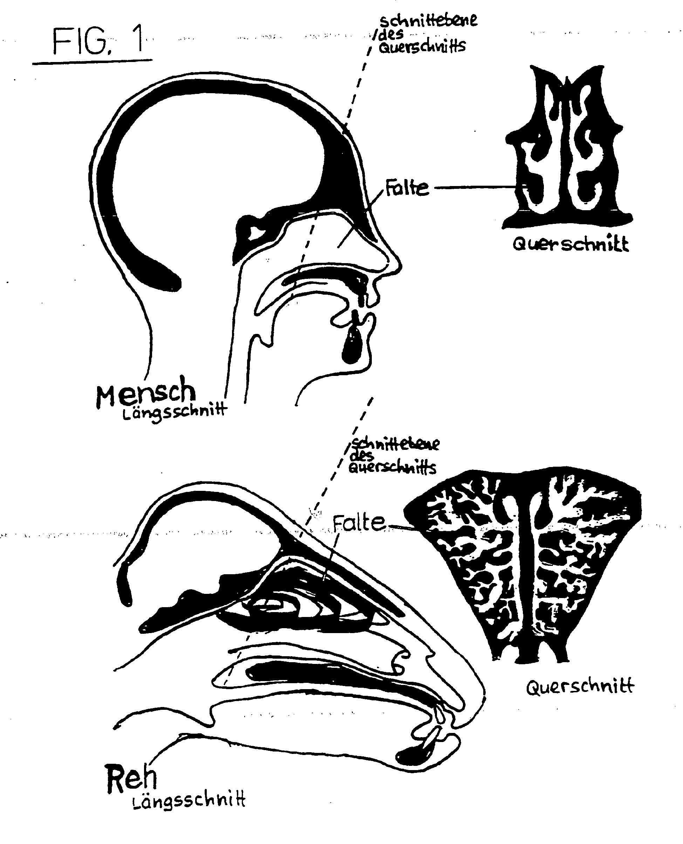

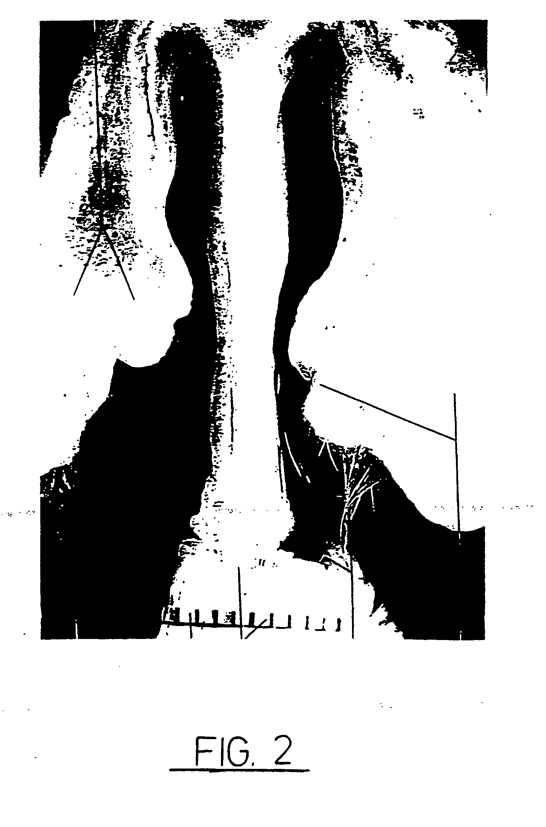

Die Schleimhaut des Mukosa-assoziierten Immunsystems befindet sich in der Nasenhöhle an der lateralen Nasenwand in Bereich der Turbinalien (Nasenmuscheln), wie beispielsweise Figur 1 zeigt, die humane Turbinalien und Turbinalien eines Rehs darstellt. Unsere Untersuchungen haben ergeben, daß mit den bisherigen Nasensprays wegen erheblicher Verluste im Nasenvorhof (Vestibulum nasi) (siehe Figur 2) sowie an der Nasenscheidewänd nur eine ungenügende Menge der pharmazeutisch aktiven. Substanz in die Nasenhaupthöhlen gelangt. Diese Nasenstrukturen sind mit immunologisch inaktivem Plattenepithel und Talgdrüsen ausgekleidet. (Walter Becker, Hans Heinz Naumann, Carl Rudolf Pfaltz: Hals-Nasen-Ohren-Heilkunde, Thieme Verlag, Stuttgart, New York 1992).

US 5,797,390 offenbart einen Nasensprayapplikator mit einem Aufsatzteil. Das Aufsatzteil ist so dimensioniert und geformt, dass es genau in die Nasenlöcher (eines Menschen bzw. eines Tieres) passt; dabei erstreckt sich ein proximales Ende des Aufsatzteils vorzugsweise nach außen, um den Benutzer daran zu hindern, das Aufsatzteil zu tief in die Nasenöffnung einzuführen.

FR 2 739 294 beschreibt eine Abgabeeinrichtung mit einer Manschette. Die Manschette dient jedoch dazu, die Länge des in die Nasenöffnung eindringenden Abschnitts der Abgabeeinrichtung gezielt zu begrenzen, um eine Verletzung der Nasenschleimhäute durch die Abgabeeinrichtung zu verhindern.

US 2,434,875 offenbart eine Sprühvorrichtung zur nasalen Verabreichung von Medikamenten, indem eine abgemessene Medikamentenmenge in einem schmalen, üblicherweise nadelförmigen Strahl in den Nasengang gesprüht wird.

EP-A-0 170 198 beschreibt einen Spender zur intranasalen Applikation von Wirkstofflösungen, der so ausgeformt ist, dass der Anwender notwendigerweise seinen Kopf nach hinten neigt und so die aus medizinischer Sicht gewünschte Kopfhaltung einnimmt.

WO 98/57690 offenbart einen Nasensprayapplikator, der eine Manschette beinhaltet, die bei Betätigung zumindest teilweise in ein Nasenloch eingeführt wird. Der Nasensprayapplikator kann auch ein Verbindungsstück aufweisen, welches auf den Nasensprayapplikator in ein einer definierten Ausrichtung aufgesteckt werden kann, sowie ein Abstützvorrichtung, die eine kontrollierte Ausrichtung des Applikators während der Verwendung gewährleistet.

Ursachen für das Unvermögen bisheriger Nasenapplikatoren, eine suffiziente Dosis auf die respiratorische Schleimhaut (Ort der spezifischen und unspezifischen Abwehr) zu versprühen, sind verschiedener Art:
(1) Aufgrund ungenügender ungenügender Anatomiekenntnisse wird der Hauptvektor des Sprühstrahls bei der Sprayapplikation in eine falsche Richtung gelenkt. Dabei gelangt die pharmazeutisch aktive Substanz kaum in die Nasenhaupthöhle. Die versprühte Flüssigkeit fließt teilweise über die Oberlippe nach unten heraus.
(2) Der Sprühansatz beim Nasenteil ist in seinem Durchmesser nicht den anatomischen Verhältnissen angepaßt Das innere Nasenloch ist natürlicherweise spaltförmig und hat die Funktion einer Düse (siehe Figur 2). Die üblicherweise breiten Nasenstücke vermögen diese anatomische Enge nicht zu überwinden. Beim Versprühen vor dem Lumen nasi gelangt nur derjenige Sektor des Sprühkegels in die Nasenhaupthöhle, der der Breite des Lumen nasi entspricht.
(3) Der optimale Sprühwinkel wurde bisher noch nicht wissenschaftlich ausgelotet. Bei den herkömmlichen Sprayapplikatoren variiert dieser willkürlich zwischen 30° und 80° zur Horizontalen bei normal gehaltenem Kopf bzw. zu einer Senkrechten zur Längsachse des Körpers eines Menschen Damit sind erhebliche Verluste, nämlich bis 90% des applizierten Volumens ebenfalls erklärlich. Unsere systematischen Untersuchungen haben gezeigt, daß der optimale Sprühwinkel zwischen 50° und 80° liegt.
(4) Herkömmliche Nasensprays haben ein zu kurzes Nasenstück. Die Distanz von der Fingermanschette bis zur Sprühöffnung ist zu kurz. Man gelangt mit den bisherigen Sprays nicht einmal bis zum inneren Nasenloch. Das Nasenstück sollte mindestens 0.7 cm länger sein, besonders bevorzugt weist das Nasenstück eine Länge von 1 bis 1.5 cm auf.

Es ist daher eine Aufgabe der vorliegenden Erfindung eine verbesserte Abgabeeinrichtung sowie eine verbesserte Manschette mit einer Verbindungseinrichtung zum Anordnen an der Abgabeeinrichtung, insbesondere zur Prävention und Behandlung von Infektionskrankheiten bereitzustellen. Diese Aufgabe wird mit den Merkmalen der Patentansprüche gelöst.

Die wirksamste Zusammensetzung für die präventive und therapeutische Wirkung besteht aus der Formel des überraschend kostimulierenden Effektes von Liposomen, gemischt mit einem mukosalen Adjuvans und appliziert mit einer neuen Abgabeeinrichtung bzw. einem neuen Nasenspray.

Dementsprechend besteht der Erfindungskomplex aus folgenden Komponenten:
(a) einer aktiven Substanz (Antigen);
(b) einer ausgewogenen Mischung von Liposomen und mukosalem Adjuvans; und
(c) einem neuen medizinischen Gerät, nämlich einer spezifischen Abgabeeinrichtung bzw. Nasenspray.

Insbesondere werden Vakzine zur intranasalen Applikation beschrieben, bestehend aus:
(a) Influenzaoberflächenproteinen, welche liposomal formuliert sind (Virosomen);
(b) mukosalem Adjuvans bakteriellen Ursprungs; und
(c) spezifischem Sprayapplikator, der so konstruiert ist, daß nahezu 100% des Spraystosses vollumfänglich auf die für die Wirksamkeit wichtige Nasenschleimhaut appliziert werden kann.

Die aktive Substanz bezieht sich daher auf Antigene, insbesondere auf Influenzaglykoproteine, welche wegen ihrer transmembranen Domänen leicht in künstliche Membranen (Liposomen) eingebaut werden können. Es können jedoch leicht noch weitere Antigene allein oder in Kombination mit den Influenzaantigenen auf die Oberfläche der Liposomen gekoppelt werden. Die Koppelung geschieht je nach chemischer Beschaffenheit der Antigene spontan oder mittels eines chemischen Crosslinkermoleküls, wie dies schon früher beschrieben worden war (Martin FJ, Papahadiopoulos D: Irreversible coupling of immunoglobulin fragments to preformed vesicles. J. Biol. Chem. 257 (1982) 286-288). Es kann sich auch um ein DNS-Plasmid oder ein RNS-Plasmid handeln, welches für ein Antigen kodiert und in die Liposomen eingeschlossen werden kann.

Der Begriff "Antigen" in Sinne dieser Erfindung umfaßt sowohl vollständige Moleküle als auch Fragmente dieser Moleküle, die antigene Eigenschaften haben und/oder zur Immunisierung eingesetzt werden können. Weiterhin umfaßt der Begriff "Antigen" Moleküle und/oder Fragmente von Molekülen, die immunstimulierend sind.

Das in der erfindungsgemäßen Vakzine eingesetzte Influenzaoberflächenprotein umfaßt vorzugsweise Hämagglutinin (HA). Weiterhin kann das Influenzaoberflächenprotein Hämagglutinin (HA) in Verbindung/Kombination mit Neuraminidase (NA) umfassen.

In einer besonderen Ausführungsform betrifft die Erfindung eine Vakzine, welche weitere Antigene enthält. Insbesondere können die weiteren Antigene an der Oberfläche der Virosomen gebunden sein.

In einer weiteren bevorzugten Ausführungsform betrifft die Anmeldung erfindungsgemäße Vakzine, die neben den Antigenen bestehend aus Influenzaoberflächenprotein(en) weitere Antigene, vorzugsweise von pathogenen Organismen enthalten. Hierbei kann der pathogene Organismus ein Virus, ein Bakterium, ein Pilz oder ein Parasit sein. Diese Organismen umfassen verschiedene Krankheitserreger wie, z.B.: Hepatitis A-Virus, Hepatitis B-Virus, respiratorisches Syncytial-Virus (Pneumovirus), Parainfluenzavirus, Mumpsvirus, Mobillivirus, HIV, Diphterie-Bakterium (Corynebacterium diphtheriae), Tetanusbazillus (Clostridium tetani), Pneumokokken, Haemophilus influenzae, E. Coli, Candida albicans, Candida tropicalis, Candida pseudotropicalis, Candida parapsilosis, Candida krusei, Aspergillus-Arten, Trichomonas-Arten, Trypanosoma-Arten, Leishmania-Arten, Toxoplasma gondii, Plasmodium falciparum, Plasmodium vivax, Plasmodium ovale, Plasmodium malariae, Trematoden-Arten und Nematoden-Arten. Insbesonders umfassen die Trematoden-Arten Schistoma haematobium, S. mansoni, S. japoniaum und die Nematoden-Arten Tricharis trichiura, Ascaris lumbricoides und Trichinella spiralis.

Weiterhin umfaßt die Erfindung eine Vakzine, welche ein Influenzavakzine ist. Insbesondere umfaßt die Erfindung eine Vakzine, welche zur Prävention und/oder Behandlung allgemeiner Infektionen/Infektionserkrankungen angewandt werden kann. Weiterhin dient die Vakzine zur Prävention und/oder Behandlung von Influenzaerkrankungen, zur Prävention und/oder Behandlung von verstopfter Nase, zur Behandlung von Verletzungen von Nasenschleimhäuten. Weiterhin umfaßt die Erfindung vorzugsweise Vakzine, wobei der Hämagglutinin-Gehalt pro Dosis (100 µl) zwischen 1 - 30 µg, mit Vorteil zwischen 3 - 10 µg und am besten bei 3.75 µg liegt.

In einer bevorzugten Ausführungsform weist die Erfindung auf eine Vakzine, wobei das Verhältnis Liposomen-Phospholipid zu HA zwischen 1:10 und 20:1, vorzugsweise zwischen 1:1 bis 10:1 und besonders bevorzugt bei 3:1 liegt.

Desweiteren bezieht sich die Erfindung vorzugsweise auf eine Vakzine, wobei das Phospholipid der Liposomen ausgewählt ist aus der Gruppe von neutralen, kationischen und/oder anionischen Phospholipiden.

In einer bevorzugten Ausführungsform bezieht sich die Erfindung auf eine Vakzine, bei der das mukosale Adjuvans ein aktives Toxin, ein inaktives Toxin und/oder ein nicht-toxisches Toxin ist. Insbesondere umfaßt das mukosale Adjuvans, vorzugsweise, *Heat Labile Toxin* (HTL), Choleratoxin (CT) und/oder Procholeragenoid (PCG). In einer anderen bevorzugten Ausführungsform ist das mukosale Adjuvans *das Heat Labile Toxin* (HLT) von *Escherichia coli.* In einer weiteren bevorzugten Ausführungsform können die Toxine inaktiviert sein. Diese Inaktivierung kann durch Rekombinationstechnologie erfolgen.

In einer weiteren Ausführungsform umfaßt die Erfindung eine Vakzine, welche *Heat Labile Toxin* (HLT) und/oder Choleratoxin (CT) in einem Verhältnis enthält, das zwischen 1:2 bis 20:1, besser zwischen 1:1 und 1:10, und am besten bei 7,5 : 1 liegt.

Der oben verwendete Begriff "ausgewogene Mischung aus Liposomen und mukosalem Adjuvans" bezieht sich einerseits auf das richtige Verhältnis von Antigen und Phospholipiden sowie mukosalem Adjuvans, das nötig ist für eine zufriedenstellende Wirksamkeit. Klinische Untersuchungen haben folgendes gezeigt: Das ideale Verhältnis Phospholipid (z. B. Phosphatidylcholin, Phosphatidylethanolamin, neutrale, anionische oder kationische Phospholipide) zu Influenza-Antigen liegt zwischen 1:1 bis 20:1. Am idealsten liegt es bei 3:1. Das Verhältnis Influenza-Antigen zum aktiven mukosalen Adjuvans (Toxin) HLT oder CT liegt zwischen 1:2 bis 20:1. Das vorteilhafteste Verhältnis liegt bei 7,5:1.

Wie oben erwähnt liegt das Verhältnis Influenza-Antigen zum inaktiven mukosalen Adjuvans (inaktives Toxin) PCG oder nicht toxischen Derivat von HLT und CT zwischen 3:1 und 1:20, idealerweise bei 1:2.

In vitro-Experimente zur Messung der immunstimulierenden mukosalen Wirkung haben überraschenderweise gezeigt, daß eine Mischung der Adjuvantien Liposomen und mukosales Adjuvans nicht eine Addition der Wirkungen bewirkt, sondern daß die Addition um einen Faktor von mindestens fünf erhöht wird.

Der Begriff "neues medizinisches Gerät" bezieht sich auf einen Spray-Applikator, der sich besonders für die intranasale Immunisierung (Vakzination) eignet. Es genügt nicht, eine mukosal wirksame Substanz zusammenzustellen, wenn die Applikation schlecht oder ungenügend ist.

Das neue Gerät zeichnet sich dadurch aus, daß es die anatomischen Gegebenheiten der Nase vollständig berücksichtigt:
(1) Die Distanz von Fingermanschetten bis Sprühkopf beträgt vorzugsweise mindestens 4,0 cm.
(2) Der vordere Teil des Nasenstücks besteht aus einem im wesentlichen zylindrischen Aufsatz, der beispielsweise mindestens 5 mm lang und höchstens 5 mm im Durchmesser ist.
(3) Der Sprühwinkel der in dieser Erfindung beschriebenen Abgabeeinrichtung beträgt vorzugsweise 50° bis 70°zur Horizontalen.
(4) Die Achse der Hauptrichtung des Abgabeeinrichtung bzw. des Sprayapplikators wird vorzugsweise durch eine spezielle Manschette bestimmt: Die Manschette ist vorzugsweise auf das Vorderteil des Nasenstücks der Abgabeeinrichtung aufsteckbar und derart ausgebildet, daß sie bei der nasalen Sprayapplikation auf der Oberlippe abgestützt werden kann, so daß die Sprührichtung im wesentlichen zur lateralen Wand der Nasenhöhle zielt.

Eine Ausbildung der Manschette zur Abstützung auf der Oberlippe ist bevorzugt, da die Oberlippe relativ nahe an der Nase liegt und dennoch die Distanz groß genug ist, um einen wirksamen Hebel zur vorteilhaften Ausrichtung zu bilden. Außerdem ermöglichen die sensiblen Tastsensoren der Lippe dem Benutzer, die richtige (zentrische bzw. parallele) Auflage der Manschette, ohne einen Blick in den Spiegel zu überprüfen. Daneben kann die Manschette jedoch auch zur Abstützung an anderen Körper-bzw. Kopfpartien ausgebildet zu sein.

Das schmale Nasenstück hat vorzugsweise eine Mindestlänge von 0.5 cm, besser 1 cm, und das nachfolgende dickere Nasenstück ist bevorzugt mindestens 1 cm, mit Vorteil 2 cm, lang. Die Distanz zwischen dorsaler Fingeroberfläche und Sprayspitze sollte mindestens 2 cm, mit Vorteil 2,5 bis 3 cm, betragen bzw. die Distanz von Fingerabsatz (Manschette) bis Sprayspitze sollte mindestens 3 cm, mit Vorteil 4 bis 5 cm betragen. Der Sprühwinkel des Sprays sollte vorzugsweise 50° bis 70° zur Horizontalen betragen, wenn der Kopf in einer normalen senkrechten Position gehalten wird.

Zudem sollte der Hauptvektor des Sprühstoßes zwischen mittlerer und unterer Nasenmuschel gerichtet sein. D.h., er soll auf den mittleren Nasengang gerichtet sein. Dies setzt in der Regel eine beinahe horizontale Richtung des Applikators voraus. Eine solche kann, wie in der vorliegenden Erfindung beschrieben, durch das Anbringen einer speziellen Applikator-Mansehette fixiert werden. Es ist auch möglich, die Manschette und den Applikator einstückig oder integral auszubilden. In diesem Fall ist die Befestigungseinrichtung/Verbindungseinrichtung fest mit dem Applikator verbunden oder einstückig mit ihm ausgeführt.

In einer bevorzugten Ausführungsform handelt es sich beim Sprayapplikator um einen Apparat, dessen Nasenstück mit Sprühkopf vorzugsweise höchstens 5 mm dick und mindestens 5 mm, besser 7 mm, lang ist, dessen Distanz zwischen Fingermanschette bis Sprühkopfspitze mindestens 3 cm beträgt und der mit einer montierbaren Manschette das Einführen in das Nasenloch so erlaubt, daß der Hauptsektor des Nasensprays mit der Horizontalen einen Winkel von ca. 15 bis 20° bildet.

In einer weiteren bevorzugten Ausführungsform ist das Antigen ein Gemisch aus Influenza-Oberflächenantigenen, das an der Oberfläche von Liposomen präsentiert wird. Dieses Mittel kann mit Antigenen von anderen pathogenen Mikroorganismen kombiniert werden. Wie oben bereits erwähnt kann die erfindungsgemäße Vakzine daher weitere Antigene, vorzugsweise Antigene anderer pathogener Organismen enthalten.

In einer weiteren Ausführungsform dient das vorstehend beschriebene Verfahren zur Prophylaxe von Infektionskrankheiten, zur Behandlung einer verstopften Nase und verschiedener Störungen der Nasenschleimhaut.

Im folgenden werden bevorzugte Ausführungsformen der Erfindung unter Bezugnahme auf die Zeichnungen beispielhaft beschrieben. Es zeigen:
- Fig. 1: eine Vergleichsdarstellung zwischen humanen Turbinalien und Turbinalien eines Rehs;
- Fig. 2: eine schematische Darstellung des anatomischen Baus eines inneren Nasenlochs;
- Fig. 3: eine schematische Darstellung des prinzipiellen Aufbaus einer bevorzugten Ausführungsform der erfindungsgemäßen Abgabeeinrichtung;

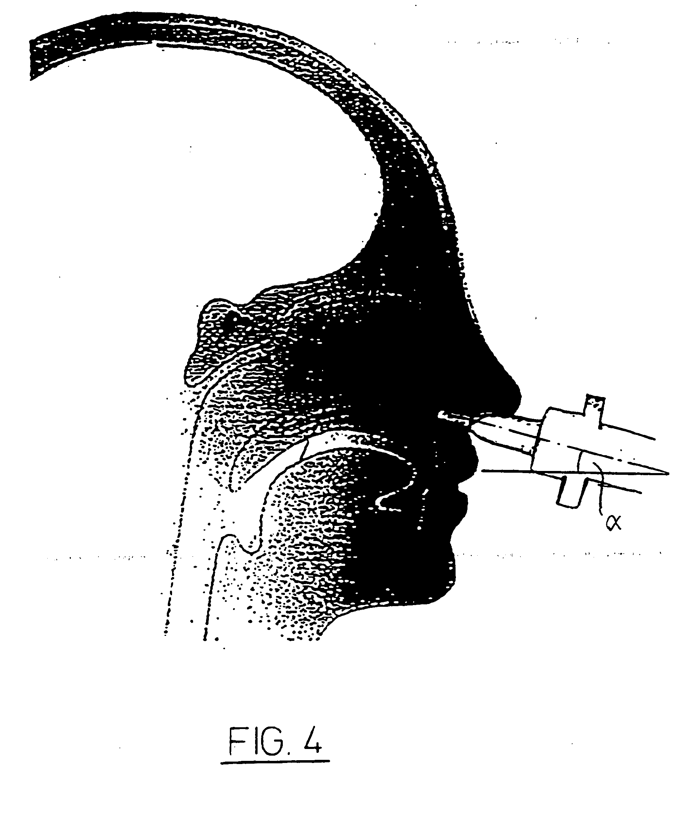

- Fig. 4: eine Darstellung des korrekten Sprühwinkels bei wirksamer Applikation;
- Fig. 5: eine Darstellung einer Nasenzytologie: Quantifizierung der verschiedenen Zellpopulationen, die durch Nasenabstrichtechnik von den Individuen (20 jeder Gruppe) bis zu 29 Tage nach drei verschiedenen Arten der intranasalen Vakzination erhalten wurden. Man beachte den deutlichen Anstieg der Centroblasten als Anzeichen einer lokalen Immunantwort in Gruppe A im Gegensatz zum signifikant geringeren Anstieg in den Gruppen B und C (p<0,005);
- Fig. 6: eine bevorzugte Ausführungsform der erfindungsgemäßen Abgabeeinrichtung ohne Manschette; und
- Fig. 7a und 7b: zwei Ansichten einer bevorzugten Ausführungsform einer Manschette für die erfindungsgemäße Abgabeeinrichtung von Fig. 6.

### BEISPIEL 1

### Herstellung einer mukosalen virosomalen Influenzavirusvakzine mit HLT als zusätzlichem mukosalen Adjuvans

Die Herstellung einer Influenzavirosomenvakzine ist beschrieben in Glück R, Wegmann A: Liposomal presentation of influenza antigens. In: Nicholson KG, Webster RG, Hay AJ, Hrsg., Textbook of Influenza (1998) S. 400-409. London: Blackwell. In Kürze umfaßt die Herstellung: Die Stämme H1 N1 A/Singapur/6/86, H3N2 A/Wuhan/359/95 und B/Beijing/184/93 des Influenzavirus, die in angebrüteten, Hühnereiern kultiviert wurden, wurden vom National Institute of Biological Standards and Control, London, GB, geliefert. Intakte Virionen wurden aus der Chorioallantois-Flüssigkeft durch Zonenzentrifugation isoliert und mit β-Propiolacton inaktiviert. Gereinigte Virionen wurden in einen Puffer gegeben, der 0,1 M Octaethylenglycolmono(N-dodecyl)ether (OEG) (Nikko Chemicals, Japan) in PBS-NaCl enthielt. Diese wurden 20 Minuten bei 21°C inkubiert, um die vollständige Zersetzung der Viruskomponenten zu ermöglichen.

Zur Extraktion von Hämagglutinin (HA) und Neuraminidase (NA) wurde das Gemisch 60 Minuten bei 100000xg zentrifugiert. Der Überstand, der. HA, NA und virale Phospholipide (PL) enthielt, wurde zur Herstellung der verschiedenen intranasalen Vakzinformulierungen verwendet. Zusätzliche Phospholipide (Phosphatidylcholin [Lipoid, Deutschland]) wurden zugegeben und löslich gemacht. Die Virosomen wurden spontan während der Entfernung des OEG-Detergens durch Chromatographie gebildet. Zu einer mukosalen Vakzindosis (100 µl), die 3,75 µg HA von jedem von drei influenzavirusstämmen, wie von der WHO empfohlen, und 35 µg Lecithin enthielt, wurden 0,5 µg *Heat Labile Toxin* (HLT) von E. coli aus dem Produktionsstamm E. coli HE22VK (Vogel FR, Powell MF: A compendium of vaccine adjuvants and excipients. In: Vaccine Design: The Subunit and Adjuvant Approach (M. F. Powell, M. J. Newmann, Hrsg.), Plenum Press, New York (1995) S. 141) als mukosales Adjuvans gegeben.

### Beispiel 2

Die E.coli-Zellen werden in einer Durchlaufzentrifuge (Westfalia AG) sedimentiert und in "Phosphate buffered saline" (PBS, 6.06 g/L Na₂HPO₄, 1.46 g/L KH₂PO₄ 2.4 g/L NaCl pH 7.4) suspendiert.

Die Freisetzung des intrazellulären hitze-labilen Toxins (HLT) erfolgt durch Zellaufschluß in einer Kugelmühle (z.B. Dyna-Mill, W.Bachofen AG) oder in einer French-Press. Zum Beispiel: 10 L Zellsuspension wird mit einer Flußrate von 33 mL/min. durch die Kugelmühle gepumpt. Die Kugelmühle ist mit 500 mL Glaskugeln gefüllt, rotiert mit 3000 min⁻¹ und die Schlitzöffnung beträgt 0.05 mm.

Die Abtrennung fester Zellbestandteile in der Zellaufschluß-Lösung erfolgt mittels tangentialer Mikrofiltration. Zum Beispiel: Die 10 L-Zell-Lysat werden in einem Prostak-System (Millipore AG) unter Verwendung eines Filters mit 0.2 µ Porengrösse auf ca. 4 L eingeengt. Danach wird mit 24 L PBS nachgespült. Das Permeat wird durch einen 0.2 µ-Sterilfilter (z.B. Gelman Supor DCF, Pall) filtriert.

Aus dem Permeat wird das HLT mittels Affinitäts-Chromatographie isoliert. Als stationäre Phase wird immobilisierte Galaktose (Galaktose-Gel z.B. von Pierce) oder immobilisierte Laktose (Laktosyl-Gel z.B. Pharmacia) verwendet. Die mobile Phase besteht aus PBS (Puffer A) und 5% (g/v) Laktose in halb konzentriertem PBS (Puffer B). Zum Beispiel: Das sterilfiltrierte Zell-Lysat wird auf die mit Puffer A vorkonditionierte Säule aufgetragen und die Säule danach mit Puffer A gespült, bis die UV-Absorption bei 280 nm die Basislinie erreicht. Danach wird das HLT mit Puffer B von der Säule eluiert.

### BEISPIEL 3

### Herstellung einer mukosalen virosomalen Influenzavakzine mit PCG als zusätzlichem mukosalen Adjuvans

Die Herstellung einer Influenzavirosomvakzine ist im Beispiel 1 beschrieben. Zu einer mukosalen Vakzindosis (100 µl), die 3,75 µg HA von jedem von drei Influenzavakzinstämmen, wie von der WHO empfohlen, und 35 µg Lecithin enthielt, wurden 6 µg Procholeragenoid, hergestellt durch Erhitzen von gereinigtem CT, das aus V. cholerae (Inaba 569B) stammte, als mukosales Adjuvans gegeben (Pierce NF, Cray WC, Sacci JB, Craig JP, Germanier R. Fürer E: Prochoteragenoid : A safe and effective antigen for oral immunization against experimental cholera. Inf. Immunity 40, 3 (1983) 1112-1118).

### BEISPIEL 4

### Herstellung einer mukosalen Hepatitis-B-(HBs-)Vakzine

Phosphatidylethanolamin (R. Berchtold, Biochemisches Labor, Bern, Schweiz) wurde in Methanol gelöst, und 0,1 % (Vol./Vol.) Triethylamin wurde zugegeben. Die Lösung wurde dann mit γ-Maleimidobuttersäure-N-hydroxysuccinimidester (GMBS) (Pierce Chemical Company, Rockford, IL) (Verhältnis Phosphatidylethanolamin:GMBS = 2:1) gemischt, das zuvor in Dimethylsulfoxid (DMSO) gelöst worden war. Nach 15-minütiger Inkubation bei Raumtemperatur wurden die Lösungsmittel 1 Stunde unter Vakuum in einer Speedvac-Zentrifuge verdampft. Rekombinante HBs-Partikel wurden gemäß bekannter Verfahren in CHO-Zellinien hergestellt und gereinigt.

Um ein reduziertes HBs-Partikel mit freien Cysteinresten zu erhalten, wurden die Partikel mit 40 mMol/l DL-Dithiothreitol (DTT) für 5 Minuten bei Raumtemperatur behandelt. Das DTT wurde unter Verwendung einer Sephadex-G10-Säule (Pharmacia LKB Biotechnology, Uppsala, Schweden) entfernt, und Octaethylenglycol (Fluka Chemicals, Schweiz) (OEG) wurde in einer Endkonzentration von 100 mMol/l zugegeben. Das verdampfte Phosphatidylethanotamin-GMBS wurde dann mit der HBs-Lösung 1 Stunde gemischt (Verhältnis HBs:GMBS = 1:2), ungebundenes GMBS wurde dann durch Cystein abgefangen. Die Umsetzungen wurden mittels Dünnschichtchromatographie überwacht.

32 mg Phosphatidylcholin (Lipoid GmbH, Ludwigshafen, Deutschland) und 6 mg Phosphatidylethanolamin wurden zu den zuvor vemetzten Phosphatidylethanoiamin-GMBS-HBs gegeben, und dieses Gemisch wurde in einem Gesamtvolumen von 2,66 ml PBS, die 100 mM OEG enthielt (PBS-OEG), gelöst.

Das Influenza A/Singapur-Hämagglutinin wurde gereinigt, wie vorstehend im Beispiel (1) beschrieben. Eine Lösung, die 4 mg Hämagglutinin enthielt, wurde 30 Minuten bei 100000g zentrifugiert, und das Sediment wurde in 1,33 ml PBS-OEG gelöst.

Die Phospholipide und die Hämagglutininlösung wurden gemischt und 1 Minute mit Ultraschall behandelt. Das Gemisch wurde dann 1 Stunde bei 100000g zentrifugiert und der Überstand sterilfiltriert (0,22 µm).

Virosomen mit adsorbiertem HBs wurden dann durch Entfernung des Detergens unter Verwendung von BioRad-SM-Bio-Beads gemäß Beispiel 1 entfernt. Einer Stammlösung enthaltend 50 µg HBs-Antigen/ml wurde 10 µg/ml HLT aus Beispiel 2 Zugegeben und in Sprayapplikatoren gemäß Beispiel 6 abgefüllt.

### BEISPIEL 5

### In-vitro-Test zum Testen der Aktivität des mukosalen Adjuvans

Die biologischen Aktivitäten der mukosalen Adjuvantien wurden mit Influenzavirosomen, HLT, PCG, einem Gemisch von Virosomen und HLT gemäß Beispiel 1 und einem Gemisch von Virosomen und PCG gemäß Beispiel (2) gemessen. Die nachstehenden Lösungen wurden zu Y-1-Nebennierenzellen ATCC: CCL 79 (Y-1-adrenal tumor, mouse steroid secreting) in Minikultur gemäß Sack DA und Sack RB (Sack DA und Sack RB: Test for enterotoxigenic Escherichia coli using Y-1 adrenal cells in miniculture. Infect. Immun. 11 (1974) 334-336) gegeben:
(a) HLT (5 µg/ml), (b) PCG (60 µg/ml), (c) Influenzavirosomen und HLT: 37 µg/ml Influenzahämagglutinin von jeweils 3 Stämmen (H1N1, H3N2, B), 100 µg/ml Phosphatidylcholin und 5 µg/ml sowie (d) Influenzavirosomen und PCG: 37 µg/ml Influenzahämagglutinin von jeweils 3 Stämmen, 100 µg/ml Phosphatidylcholin und 60 mg/ml PCG.

Die nachstehenden Adjuvansaktivitäten (in Einheiten) wurden bestimmt:
(a) 15 Einheiten, (b) 6 Einheiten, (c) 80 Einheiten, (d) 36 Einheiten. Die höchste biologische Adjuvansaktivität wurde mit dem Gemisch aus Virosomen und HLT oder Virosomen und PCG durchgeführt. Die Adjuvanswirkung wurde mittels Zelltoxizitätsparametern bestimmt.

### BEISPIEL 6

### Klinische Bewertung verschiedener Sprayapplikatoren

Die erfindungsgemäße Abgabeeinrichtung ist in ihrem Grundaufbau im wesentlichen mit herkömmlichen Sprayapplikatoren vergleichbar, ist jedoch besonders gut an die anatomischen Gegebenheiten für eine nasale Applikation einer beliebigen, insbesondere jedoch der hierin beschriebenen pharmazeutisch wirksamen Substanzen, angepaßt.

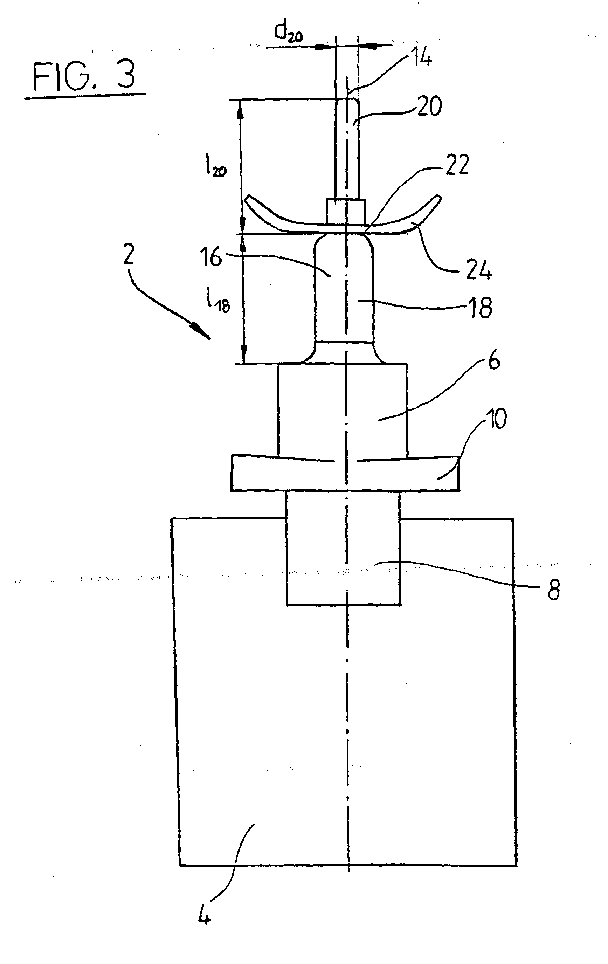

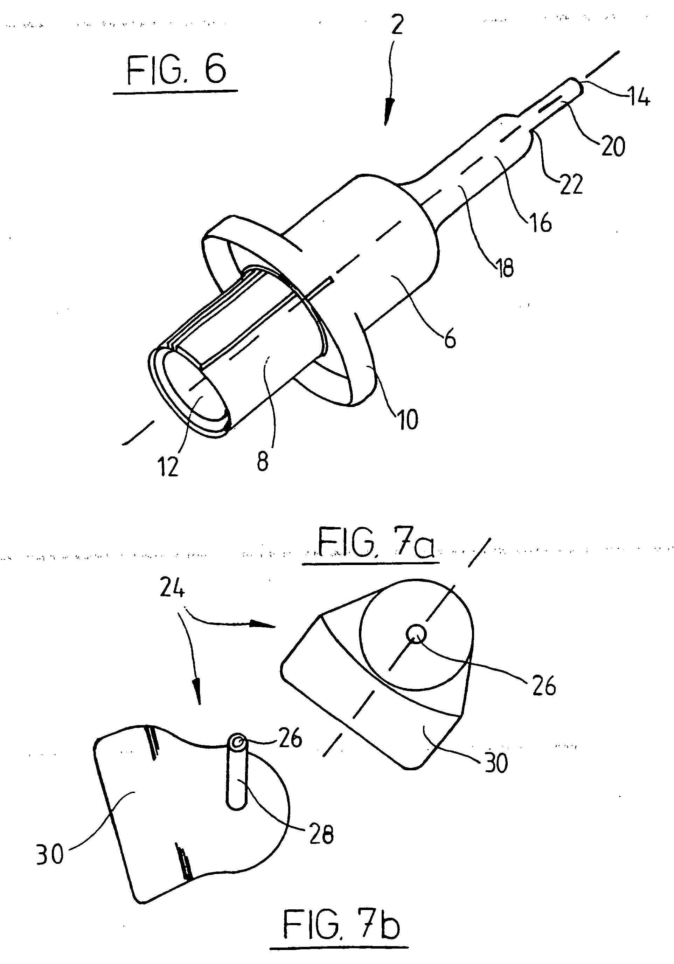

Eine bevorzugte Ausführungsform der erfindungsgemäßen Abgabeeinrichtung 2 ist in den Figuren 3 und 6 gezeigt. In der in Figur 3 dargestellten Seitenansicht der erfindungsgemäßen Abgabeeinrichtung (Sprayapplikator) 2 sind die wesentlichen Komponenten erkennbar. Die Abgabeeinrichtung 2 weist im wesentlichen einen Behälter 4 mit einer (nicht dargestellten) pharmazeutisch wirksamen Substanz und einen Spray- bzw. Pumpmechanismus 6 auf. Der Pumpmechanismus 6 ist mit einem Verbindungsabschnitt 8 fluiddicht mit dem Behälter 4 verbunden. Das Pumpen und Sprühen mit der erfindungsgemäßen Abgabeeinrichtung 2 erfolgt auf herkömmliche Weise durch Betätigung einer Fingermanschette 10. Die im Behälter 4 befindliche pharmazeutisch wirksame Substanz wird durch eine sich durch den Pumpmechanismus erstreckende Öffnung 12 an einer Austrittsöffnung 14 abgegeben.

Die erfindungsgemäße Abgabeeinrichtung 2 unterscheidet sich von den bekannten Sprayapplikatoren insbesondere durch die konstruktive Ausgestaltung des Nasenstücks 16, das beim effektiven Applizieren der pharmazeutisch wirksamen Substanz eine wesentliche Rolle spielt. Das Nasenstück 16 ist vorzugsweise integral mit dem Pumpmechanismus 6 und der Fingermanschette 10 ausgebildet. Das Nasenstück 16 ist vorzugsweise in zwei Abschnitte 18 und 20 unterteilt, wobei der in Richtung der Fingermanschette 10 gelegene Abschnitt 18 einen größeren Durchmesser aufweist als der benachbart zur Austrittsöffnung 14 gelegene Abschnitt 20 des Nasenstücks 16. Der Übergangsbereich zwischen den beiden Abschnitten 16 und 20 bildet einen Anschlag 22 für eine - gemäß einer bevorzugten Ausführungsform der vorliegenden Erfindung - am Abschnitt 20 des Nasenstücks 16 befestigbare Manschette 24 zur Abstützung an der Oberlippe eines Benutzers.

Der zur Austrittsöffnung 14 benachbart gelegene Abschnitt 20 des Nasenstücks 16 ist vorzugsweise im wesentlichen zylindrisch ausgebildet und weist einen Durchmesser d₂₀ von maximal 5 mm, bevorzugt etwa zwischen 2 und 4 mm auf. Die Länge l₂₀ des vorderen Abschnitts 20 des Nasenstücks 16 beträgt beispielsweise mindestens 5 mm, bevorzugt mindestens 10mm und besonders bevorzugt 10 bis 20 mm. Die Manschette 24 ist über den Abschnitt 20 des Nasenstücks 16 vorzugsweise von der Austrittsöffnung 14 her aufschiebbar und bevorzugt mittels einer Verdrehsicherung drehfest auf der Abgabeeinrichtung 2 gehalten. Die Verdrehsicherung kann beispielsweise in Form eines Polygonprofils, z.B. mit elliptischem Querschnitt, am vorderen Abschnitt 20 des Nasenstücks 16 und einer entsprechenden Aussparung 26 in der Manschette 24 realisiert sein.

Entsprechend einer anderen Ausführungsform der erfindungsgemäßen Abgabeeinrichtung 2 kann der in die Nase eines Patienten ragende Abschnitt des Sprayapplikators auch an der Manschette 24 ausgebildet sein. Eine derartige Ausführungsform ist in Figur 7b beispielhaft dargestellt. Wie in Figur 7b gezeigt, ist an der Manschette 24 ein im wesentlichen rohrförmiger Absatz 28 ausgebildet, der auf den zur Austrittsöffnung 14 benachbart gelegenen Abschnitt 20 des Nasenstücks 16 aufsetzbar ist. Die Abmessungen des rohrförmigen Abschnitts 28 entsprechen in diesem Fall bevorzugt im wesentlichen den Abmessungen (d₂₀ und l₂₀) der vorstehend beschriebenen Ausführungsform.

Der Abschnitt 18 des Nasenstücks 16 weist vorzugsweise eine Länge I₁₈ von mindestens 10 mm und bevorzugt etwa 20 mm auf. Die Abmessungen der beiden Abschnitte 18 und 20 werden vorzugsweise so gewählt, daß die Distanz zwischen der dorsalen Fingeroberfläche eines Benutzers und der Sprayspitze bzw. Austrittsöffnung 14 mindestens 2 cm, vorteilhafterweise jedoch 2,5 bis 3 cm beträgt. Dies ist insbesondere dann gewährleistet, wenn die Distanz zwischen der Manschette 24 bis zur Sprayspitze 14 mindestens 3 cm oder vorteilhafterweise 4 bis 5 cm beträgt.

Durch die erfindungsgemäße Abgabeeinrichtung 2 mit diesen Abmessungen kann die pharmazeutisch wirksame Substanz wesentlich zielgenauer in die Nasenhaupthöle bzw. in das innere Nasenloch gesprüht werden, so daß eine erheblich höhere Wirksamkeit als mit bekannten Systemen erzielbar ist.

Die erfindungsgemäße Abgabeeinrichtung 2 ist vorteilhafterweise mit der Manschette 24 versehen, so daß zusätzlich zu den günstigeren Abmessungen ein optimaler Sprühwinkel α einstellbar ist. Die Manschette 24 ist jedoch auch unabhängig von dem vorstehend beschriebenen Sprayappliktator 2 an herkömmlichen Abgabeeinrichtungen vorsehbar, so daß auch mit diesen herkömmlichen Sprayapplikatoren aufgrund des genau einstellbaren Sprühwinkels α eine erheblich verbesserte Wirksamkeit erzielt werden kann.

Die erfindungsgemäße Abgabeeinrichtung 2 kann folglich entweder durch die Wahl der Abmessungen des Nasenstücks 16 oder durch eine geeignete Manschette 24 zur Einstellung eines optimalen Sprühwinkels α die verbesserte Wirksamkeit erzielen. Bevorzugt ist jedoch eine Kombination der neuartigen Abgabeeinrichtung 2 mit den vorstehend beschriebenen Abmessungen und der Manschette 24 zur Einstellung eines optimalen Sprühwinkels α um bestmögliche Behandlungsergebnisse zu erzielen.

Die Manschette 24 wird wie bereits vorstehend kurz erläutert mit dem rohrförmigen Abschnitt 28 über den Abschnitt 20 des Nasenstücks 16 geschoben. Im montierten Zustand ist die Manschette 24 vorzugsweise drehfest mit der Abgabeeinrichtung 2 verbunden. Beispielsweise kann die Verdrehsicherung durch ein an zumindest einer Seite abgeflachtes Kreisprofil am Nasenstück 16 sein, mit dem die Manschette 24 mittels einer entsprechend geformten Öffnung drehfest montierbar ist. Die Manschette 24 weist einen Abstützabschnitt 30 auf, der an der Oberlippe eines Benutzers anlegbar ist, um zusammen mit dem in das Nasenloch eines Patienten weisenden Abschnitt 20 des Nasenstücks 16 den Sprühwinkel α definiert festzulegen. Der optimal Sprühwinkel α liegt vorzugsweise zwischen 50° und 70 °.

Der Abstützabschnitt 30 der Manschette 24 ist vorzugsweise korrespondierend zur Form der Oberlippe leicht gebogen, um eine optimale Anlage- und Abstützfläche bereitzustellen. Die Manschette 24 kann aus jedem geeigneten Material hergestellt sein, bevorzugt sind jedoch spritzgießbare Kunststoffmaterialien.

Zur Bewertung der Wirksamkeit wurden verschiedene Abgabeeinrichtungen getestet. Dazu wurde 1% Methylenblaulösung in fünf verschiedene Typen von nasalen Applikatoren eingefüllt:
(a) Ein Sprayapplikator gemäß dieser Erfindung mit Richtmanschette, welche die Richtung des Spraystoßes fixiert;
(b) ein Sprayapplikator gemäß dieser Erfindung ohne Richtmanschette;
(c) ein herkömmlicher kommerzieller Sprayapplikator ohne schmales Nasenstück von 2 cm Länge und 4 mm Durchmesser mit einem Sprühwinkel von 50°;
(d) ein Sprayapplikator gemäß dieser Erfindung, jedoch mit einem Sprühwinkel von 50°; und schließlich.
(e) ein Sprayapplikator gemäß dieser Erfindung, jedoch mit Fingermanschetten, die lediglich 3 cm vom Sprühkopf entfernt waren.

Sämtliche Sprayapplikatoren waren sog. Bidosis-Applikatoren. Jeder Sprayapplikator wurde von je 5 Versuchspersonen unter ärztlicher Aufsicht ausprobiert. Die Versuchspersonen sprayten je eine Dosis Methylenblaulösung in das eigene rechte und linke Nasenloch. Mittels Nasenendoskopie wurden die Mukosa-Turbinalien ausgeleuchtet und photographisch festgehalten. Die Intensität der Blaufärbung sowie die blau gefärbte Fläche auf den Turbinalien wurden mittels einer Skala von 1 - 4 ausgewertet. Die Auswertung ergab folgendes Resultat (geometrisches Mittel):

Gruppe (a): 19 Punkte, Gruppe (b): 16 Punkte, Gruppe (c): 7 Punkte, Gruppe (d): 9 Punkte, Gruppe (e): 12 Punkte.

Nur in der Gruppe (a) wurde die Sprayflüssigkeit fast ausschließlich auf die Nasenschleimhaut appliziert. Auch in der Gruppe (b) wurde ein großer Teil des Sprayinhaltes auf die Schleimhaut gesprayt. In den übrigen Gruppen wurde ein wichtiger Anteil des Sprayinhaltes in den Nasenvorhof gesprayt. Dort kann die zu applizierende Sprayflüssigkeit keine Wirksamkeit ausüben.

### BEISPIEL 7

### Vergleich der konischen Wirksamkeit von intranasalen virosomalen Influenzavakzinen mit und ohne HLT, angewendet in einem neuen Spraygerät, das in der vorliegenden Erfindung beschrieben ist, in menschlichen Freiwilligen verglichen mit einer parenteralen kommerziellen Influenzavakzine

Die offene randomisierte klinische Studie wurden in völliger Übereinstimmung mit den Prinzipien der Erklärung von Helsinki und mit den örtlichen Gesetzen und Richtlinien bezüglich klinischer Studien durchgeführt. Nach der Genehmigung des Protokolls durch das Ethikkomittee des Kantons Luzem und der Bekanntgabe an die Schweizer Bundesgesundheitsbehörde gaben 80 gesunde Freiwillige (18-64 Jahre alt) ihre schriftliche informierte Einwilligung zur Teilnahme. Freiwillige wurden ausgeschlossen, wenn sie Anzeichen für eine akute oder chronische Erkrankung zum Zeitpunkt der Immunisierung aufwiesen oder wenn eine gleichzeitige Behandlung mit immunsuppressiven Arzneimitteln oder eine bekannte Immunschwäche vorlagen.

Die intranasalen Vakzinformulierungen wurden an jede von drei Gruppen mit 20 Freiwilligen verabreicht (Tabelle 1). Die Gruppen, A und B erhielten 2 Dosen der Formulierung A bzw. B in jedes Nasenloch am Tag 1 und 2 Dosen eine Woche später. Die Gruppe C erhielt zwei Dosen doppeltkonzentrierter Formulierung A am Tag 1. Die Gruppe D wurde intramuskulär im Deltoidbereich mit der parenteralen Formulierung geimpft. Blut- und Speichelproben (Omnisal®, GB) wurden unmittelbar vor der ersten Vakzination und einen Monat nach der ersten Immunisierung (Tag 29±2) abgenommen. Aufgrund eines technischen Problems konnten nur die Speichelproben der ersten 47 Personen bewertet werden. Die Bürstenzytologie der Nasenhöhle wurde vor der Immunisierung und an den Tagen 4, 8 und 1 Monat nach der Erstimmunisierung durchgeführt.

Für die Analysen wurden die Blutproben und Speichelproben kodiert.

Die Serumimmunantwort auf die HA-Vakzinkoniponente wurde durch einen Standardtest unter Verwendung von vier Hämagglutinineinheiten der entsprechenden Antigene bestimmt. Die Seren wurden vor dem Test 30 Minuten bei 56°C behandelt. Die Titer sind als reziproker Wert der höchsten Verdünnung des Serums ausgedrückt, die die Hämagglutination vollständig verhinderte. Ein Titer von ≥ 1:40 wurde als schützend angesehen.

Gesamt- und influenzaspezifische IgA-Antikörper wurden mittels bekannter ELISA-Verfahren bestimmt (Tamura SI, Ito Y, Asanuma H et al., Cross-protection against influenza virus infection afforded by trivalent inactivated vaccines inoculated intranasally with cholera toxin B. subunit. J. Immunol. 149 (1992) 981-987). Die virusspezifischen IgA-Werte wurden als ELISA-Einheiten von spezifischen IgA pro µg Gesamt-IgA-Konzentration ausgedrückt.

Die Nasenepithelzellen wurden ausschließlich von den Maxillo-Turbinaten beider Nasenhöhlen in jeder Person mit dem gleichen Typ einer kleinen Nylonbürste gesammelt, die bei zytopathologischen Untersuchungen während der Bronchoskopie eingesetzt wird (Glück U, Gebbers J-O, Nasal cytopathology in smokers: a possible biomarker of air-pollution? Am. J. Rhinol. 10 (1996) 55-57). Die Probennahme wurde durch den gleichen Untersucher (U. G.) unter rhinoskopischer Kontrolle mit einer rotierenden und translationalen Bewegung entlang der unteren Turbinatanheftung durchgeführt. Die Zellen wurden auf einen Glasobjektträger überführt und sofort in einer Lösung von 200 ml Ethanol + 100 ml Aceton + 6 Tropfen Trichloressigsäure fixiert.

Die Papanicolaou-gefärbten Objektträger wurden durch Pathologen untersucht, die am Institut für Pathologie in Cytopathologie, Kantonshospital Luzern, ausgebildet werden und nicht über den Vakzinationszustand informiert waren.

Die durchschnittlichen Zellzahlen von Flimmerzellen, Becherzellen, Lymphozyten, Centroblasten, Neutrophilen, Eosinophilen und Schuppenepithelzellen wurden in 25 repräsentativen Bereichen pro Objekträger bei 100x bestimmt.

Die Signifikanz zwischen den Grundlinien- und Postimmunisierungstitern wurde durch den Paired-T-Test bestimmt. Unterschiede in der Fähigkeit der 4 Vakzinationen, Anti-HA-Schutzantikörper in der Studiengruppe hervorzurufen, wurden durch χ² bestimmt.

Alle während der klinischen Studie beobachteten nachteiligen Ereignisse mußten aufgezeichnet werden. Ein nachteiliges Ereignis war definiert als eine nachteilige Änderung vom Grundlinien- (Vorvakzinations-) Zustand der Personen, ungeachtet dessen, ob das Ereignis als mit der Vakzination in Zusammenhang stehend betrachtet wird oder nicht. Nachteilige Ereignisse (lokale oder systemische Reaktion), die nach der Immunisierung auftraten, wurden durch den Kliniker auf einem speziellen Berichtsformular für nachteilige Ereignisse aufgezeichnet. Die Grundlinienrate der nachteiligen Ereignisse wurde vor der Immunisierung bestimmt.

80 Personen mit einem durchschnittlichen Alter von 40 Jahren und mit vergleichbarem sozialen Status wurden für die Studie rekrutiert. 27,5% der Teilnehmer waren weiblich. Alle 3 nasalen Vakzinationszubereitungen sowie die parenteralen Vakzine wurden gut vertragen. Anamnetisch gab es keine signifikanten Unterschiede zwischen den 3 Nasenvakzinstoff-Gruppen, und alle 3 Formulierungen wurden gut vertragen. In einzelnen Fällen wurden die nachstehenden möglichen begleitenden Reaktionen berichtet: Fieber, Müdigkeit, Übelkeit, Rhinitis, verstopfte Nase und Rhinopharyngitis. Auch die parenterale virosomale Vakzine wurde äußerst gut vertragen.

Die serologische Immunantwort ist in der Tabelle 2 gezeigt. Signifikante Anstiege im Titer wurden in der Gruppe A (2 nasale Vakzinationen in 7 Tagen Abstand), Gruppe C (1 nasale Vakzination, doppelt dosiert) sowie in Gruppe D (parenterale Vakzination gegen alle 3 Virusstämme) gemessen. Die höchsten geometrischen Mittel der Antikörpertiter (GMT) wurden in den Gruppen A und D gefunden. Die Gruppe D hatte signifikant die höchsten GMT-Werte gegen den H1N1-Stamm (p≤0,05). Im Fall des H3N2-Stamms gab es keine signifikanten Unterschiede zwischen den Gruppen A und D.

Diese Gruppen reagierten signifikant besser als die Gruppen B und C. Beim B-Stamm gab es keine signifikanten Unterschiede in den Gruppen A, C und D. Diese zeigten jedoch signifikant höhere Titer als Gruppe B. Die Serumkonversionsraten waren am höchsten in den Gruppen A und D. Gewöhnlich waren sie signifikant höher als die Raten in den Gruppen B und C und erfüllten für alle 3 Stämme die serologischen Anforderungen an parenterale Influenzavakzine gemäß der Europäischen Gemeinschaft (Kommission der Europäischen Gemeinschaft, Richtlinien für Medizinprodukte in der Europäischen Gemeinschaft. Harmonisierung der Anforderungen an Influenzavakzine. (1992) S. 93-98. Luxemburg: European Community Publication Office).

Die humorale mukosale Immunantwort (Speichel) ist in der Tabelle 3 gezeigt. Die höchsten Anstiege im IgA-Titer wurden in Gruppe A gemessen. Diese waren signifikant besser als in den anderen Gruppen. Unter Berücksichtigung des Gesamt-IgA waren die GMT in Gruppe A ebenfalls am höchsten. Die Mukokonversionsrate (vierfacher Anstieg im IgA-Titer) war wiederum deutlich am höchsten in Gruppe A. Im Falle der intramuskulären Vakzination gab es nur sehr schwache Mukokonversionsraten.

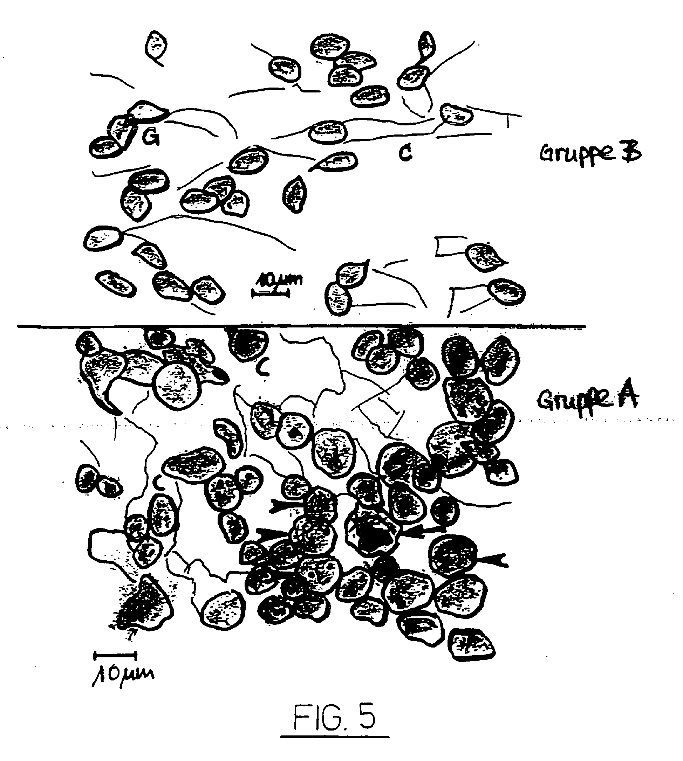

Die Bürstenzytologie der Nasenschleimhaut wurde in den Gruppen A, B und C durchgeführt. Die Ergebnisse sind in Figur 5 zusammengefaßt. Wir bestimmten die Zellzahl des Nasenschleimhautepithels (Flimmer-und Nicht-Flimmer-Säulenzellen, Becherzellen und Schuppenepithelzellen) und der Zellen der Myelo/Mono- und Lymphopoese (Lymphozyten, Eosinophile, Neutrophile und Centroblasten). In Gruppe A konnte eine deutliche Becherzellhyperplasie am 4. und 8. Tag nach der ersten Vakzination nachgewiesen werden. Außerdem beobachteten wir einen starken Anstieg an Lymphozyten und Centroblasten an den gleichen Tagen. Zusätzlich wurden ein Anstieg an Eosinophilen und Neutrophilen am 8. Tag nach der anfänglichen Vakzination beobachtet. Die Anzahl der Säulenzellen blieb unverändert.

In Gruppe B gab es nur einen leichten Anstieg an Lymphozyten, neutrophilen und eosinophilen Granulozyten. Es gab kein Anzeichen für aktivierte Lymphozyten in dieser Gruppe.

In Gruppe C wurde eine sogar noch stärkere Becherzellhyperplasie festgestellt als in Gruppe A. Außerdem waren die eosinophilen und neutrophilen Granulozyten in dieser Gruppe am stärksten an den Tagen 4 und 8 angestiegen. Der Anstieg an Lymphoblasten war in dieser Gruppe schwächer als in Gruppe A. p = ≤0,05. Einen Monat nach der ersten Vakzination war der vorherige Zustand der Zellzusammensetzung in allen Gruppen wiederhergestellt.

Fig. 5 betrifft die Nasenzytologie: Quantifizierung der verschiedenen Zellpopulationen, die durch Nasenabstrichtechnik von den Individuen (20 jeder Gruppe) bis zu 29 Tage nach drei verschiedenen Arten der intranasalen Vakzination erhalten wurden. Man beachte den deutlichen Anstieg der Centroblasten als Anzeichen einer lokalen Immunantwort in Gruppe A im Gegensatz zum signifikant geringeren Anstieg in den Gruppen B und C (p<0,005).

### BEISPIEL 8

### Vergleich der klinischen Wirksamkeit von intranasalen virosomalen Influenzavakzinen mit HLT oder PCG in menschlichen Freiwilligen, verabreicht in einem neuen Spraygerät, das in der vorliegenden Erfindung beschrieben ist

Die Immunantwort und Tolerierbarkeit von zwei Anti-Influenzasprayvakzinen wurden in einer Doppelblindstudie untersucht, die mit insgesamt 158 gesunden Schweizer Freiwilligen im Alter von 18 bis 67 Jahren durchgeführt wurde.

Eine trivalente virosomale Influenzavakzine (gereinigtes HA, formuliert mit Phosphatidylcholin) wurde mit *Heat Labile Toxin* (HLT) von E. coli in einem Format kombiniert, das sich zur intranasalen Verabreichung eignet. Eine menschliche Dosis enthielt 7,5 µg HA von jedem Influenzastamm und 2 µg HLT. Eine Dosis wurde in jedes Nasenloch an den Tagen 1 und 8 an Gruppen von Individuen im Alter von 18-59 oder ≥ 60 Jahren verabreicht. Serumproben wurden etwa 4 Wochen nach der Immunisierung entnommen. Reaktionen waren selten und schwach. Die Prozentsätze der Personen (Erwachsene bzw. Ältere), die schützende Serum-Anti-HA-Antikörpertiter erreichten (≥40) waren wie nachstehend: A/Bayern (92%, 91%), A/Wuhan (92%, 78%) und B/Beijing (59%, 50%). Die GMT nach der Immunisierung und die Vielfachen des Anstiegs der GMT waren zwischen den zwei Altersgruppen vergleichbar. Der Prozentsatz der Personen (Erwachsene bzw. Ältere), die nichtschützende Grundlinientiter besaßen, aber nach der Immunisierung schützende Spiegel erreichten, war wie nachstehend: A/Bayern (79%, 85%). A/Wuhan (86%, 56%) und B/Beijing (48%, 41 %).

Eine zweite mukosale Vakzinzubereitung enthielt 7,5 µg HA und 12 µg Procholeragenoid (PCG). Diese Zubereitung wurde ebenfalls gut vertragen und erwies sich als etwas weniger immunogen als die HLT-Zubereitung. Der Prozentsatz der Personen, die schützende Serum-Anti-HA-Antikörpertiter erreichten, war wie nachstehend : A/Bayern 94,2%, A/Wuhan 80,8% und B/Beijing 36,5%. Die Serokonversionsraten aller Gruppen sind in Tabelle 4 gezeigt.

Diese Ergebnisse zeigen, daß die auf intranasalem Weg verabreichte virosomale Influenzavakzine sicher und sehr immunogen bei erwachsenen Personen, einschließlich Älteren, ist.

### BEISPIEL 9

### Mukosale Immunantwort in Mäusen nach intranasaler Anwendung einer virosomalen Influenzavakzine mit HLT, mit PCG oder ohne zusätzliches mukosales Adjuvans

Gruppen von 10 erwachsenen weiblichen Balb/c-Mäusen wurden intranasal mit 30 µl entweder der im Beispiel (1) beschriebenen Influenzavakzine oder der im Beispiel (2) beschriebenen Influenzavakzine geimpft. Eine Kontrollgruppe von 10 Mäusen erhielt eine virosomale Vakzinzubereitung ohne zusätzliches Adjuvans, aber mit der gleichen Virosomenzusammensetzung. Eine Hälfte jeder Gruppe erhielt eine zweite intranasale Dosis eine Woche später. Nasenwäschen (NW) und Bronchoalveolarspülungen (BAL) wurden 3 Wochen später durchgeführt. Tabelle 5 zu Bsp. 9. Die Bestimmung des spezifischen IgA wurde durch ein bekanntes ELISA-Verfahren durchgeführt. Die Ergebnisse (GMT) sind in der Tabelle 6 zusammengefaßt.

Die höchsten GMT für alle drei Vakzinstämme (A/Johannesburg, A/Nanching, B/Harbin) konnten in der Gruppe nachgewiesen werden, die zweimal mit der Vakzine gemäß Beispiel (1) geimpft worden war. Diese Gruppe reagierte sogar mit den höchsten IgA-Spiegeln in BAL, nachdem sie nur einmal geimpft worden war.

Eine zufriedenstellende IgA-Antwort wurde auch in der Gruppe von Mäusen erhalten, die zweimal intranasal mit der Zubereitung von Beispiel (2) geimpft worden war. Die Kontrollgruppe, auf die die Virosomenzubereitung ohne zusätzliches mukosales Adjuvans angewendet worden war, zeigte nur eine sehr schwache mukosale Immunantwort.

Die Ergebnisse zeigen, daß in Mäusen eine intranasal verabreichte virosomale Influenzavakzine, die ein mukosales Adjuvans enthält, eine hohe mukosale Antikörperantwort induzieren konnte.

**VORKLINISCHE STUDIE DER VIROSOMALEN INFLUENZAVAKZINE IN MÄUSEN: INTRANASALE ANWENDUNG (Tabelle 5 zu Bsp. 9)**

| | | | | | | |
|---|---|---|---|---|---|---|
| | Gruppe 1 | Gruppe 2 | Gruppe 3 | Gruppe 4 | Gruppe 5 | Gruppe 6 |
| Tag 0 | Infl.- Viros.+ HLT i. n. | Infl.-Viros.+ HLT i. n. | Infl.-Viros.+ PCG i. n. | Infl.-Viros.+ PCG i. n. | lnfluenza-Virosomen | Influenza-Virosomen |
| Tag 7 | Infl.-Viros.+ HLT i. n. | - | Infl.-Viros.+ PCG i. n. | - | Infl.-Virus i. n. | - |
| Tag 28 | NW & BAL | NW & BAL | NW & BAL | NW & BAL | NW & BAL | NW & BAL |

**IgA-lnfluenza-Antikörper in Mäusen GMT der reziproken Titer (ELISA) (Tabelle 6 zu Bsp. 9)**

| Gruppen | H1N1 NW | H1N1 BAL | H3N2 NW | H3N2 BAL | B NW | B BAL |
|---|---|---|---|---|---|---|
| 1 | 320 | 12800 | 380 | 3200 | 320 | 9220 |
| 2 | neg. | 140 | Neg. | 24 | neg. | 1540 |
| 3 | 160 | 760 | 470 | 760 | 220 | 770 |
| 4 | neg. | neg. | Neg. | neg. | 10 | neg. |
| 5 | 4 | neg. | Neg. | neg. | 4 | neg. |
| 6 | neg. | neg. | Neg. | neg. | neg. | neg. |

### Beispiel 10

### Klinische Bewertung einer mukosalen Hepatitis-B- (HBs-) Vakzine

Die Vakzine wurde gemäß Beispiel 4 hergestellt und in einem NasensprayApplikator gemäß Beispiel 5 abgefüllt. Das Produkt wurde in 10 Freiwilligen getestet: Je 100 µl in jedes Nasenloch wurden an Tag 1 appliziert und eine Woche später wiederholt. Blutproben wurden am Tag 1 (vor der Impfung) sowie am Tag 29 entnommen. Die Anti-HBs-Antikörper wurden mittels RIA (Abbott) bestimmt. Das geometrische Mittel des Titers vor der Impfung war 7 IU/ml und am Tag 29 159 IU/ml.

### BEISPIEL 11

### Mukosale Immunantwort von Mäusen nach intranasaler Anwendung eines DNA-Plasmids, das für das HN-Antigen von Mumpsvirus kodiert, eingebracht in Influenzavirosomen, die 10% kationische Phospholipide enthalten, und gemischt mit HLT (5 µg/ml)

Wir immunisierten Gruppen von Mäusen intranasal mit a) nackter DNA, die für das HN-Antigen des Mumpsvirus kodierte (Gruppe C) oder b) in Virosomen eingeschlossener DNA nach Vorimmunisierung mit Virosomen (Gruppe A) oder c) ohne Vorimmunisierung (Gruppe B). Eine Kontrollgruppe (H) wurde i. n. mit lebendem Urabe-Mumpsvirus (Priorix, SKB Rixensart) immunisiert. Wie in Tabelle 7 gezeigt ist, war das geometrische Mittel des Titers (GMT) der lgG in der Gruppe von Mäusen, die die Präimmunisierung erhalten hatten (A), höher als der in den Gruppen B und C der Mäuse beschriebene (Lovell GH: Proteosomes, hydrophobic anchors, iscoms and liposomes for improved presentation of peptide and protein vaccines. In: New Generation Vaccines (1990) (G. C. Woodrow und M. M. Levine, Hrsg.) Dekker, New York, S. 141-168; Cusi MG und Glück R: Intranasal immunization of mice with mumps DNA entrapped into influenza virosomes. IBC's 4th Annual Conference on Genetic Vaccines, 25.-27. Okt. 1998, Washington D.C.). Die Gruppe von Mäusen, die i. n. mit nackter DNA immunisiert worden war, entwickelte einen sehr niedrigen IgG-Spiegel, wogegen die i. n. mit dem Mumpsvirus immunisierten Mäuse (Gruppe H) eine gute IgG-Antwort zeigten. Bei der Analyse der mukosalen Immunität fanden wir, daß alle Gruppen von Mäusen, außer den mit nackter DNA immunisierten, IgA entwickelten. Nur in den Nasenwäschen (NW) der i. n. mit dem Mumpsvirus immunisierten Mäuse konnten wir einen erhöhten Titer an IgA nachweisen (Gruppe H).

Cytokinmessungen wurden unter Verwendung von primären Milzzellen aus Mäusemilzen durchgeführt, die zwölf Tage nach der Immunisierung entnommen wurden. Die Tabelle 8 faßt repräsentative Messungen zusammen, die aus zwei getrennten Experimenten erhalten wurden. Mit Mumpsvirusantigen stimulierte Zellen aus Mäusen, die zuvor (i. n.) mit DNA-Virosomen geimpft worden waren, induzierten die Produktion von IL-2 und IFN-γ. Außerdem induzierten mit Grippe infizierte Mäuse die Produktion von IL-4. Aus den mit Mumpsvirus immunisierten Tieren entnommene Zellen produzierten IFN-γ, IL-2, IL-4 und IL-10 nach in-vitro-Stimulation mit Mumpsantigen. Die Immunisierung mit DNA-Virosomen wie die Kontrollimmunisierung mit den gereinigten Mumpsantigenen korrelierten mit dem Th1-Phänotyp. Außerdem dominierte, berücksichtigt man das Verhältnis zwischen dem IgG-Gesamtspiegel und dem virusspezifischen IgG1 oder IgG2a, die Menge an IgG2a-Isotyp in der Gruppe A, was eine Th2-Antwort anzeigt.

**Tabelle 7: Das geometrische Mittel der Titer (GMT) von humoralen IgG, IgA in der Bronchoalveolarspülung (BAL) und IgA in der Nasenwäsche bei Mäusen**

| MÄUSE | IgG | BAL IgA | NW IgA |
|---|---|---|---|
| Gruppe A | 356 | 8 | 10 |
| Gruppe B | 15 | 11 | 10 |
| Gruppe C | 12 | 2 | 2 |
| Gruppe H | 1585 | 2 | 20 |

**Tabelle 8: Repräsentationsmessungen der Cytokinproduktion in Mäusen, erhalten aus zwei getrennten Experimenten**

| Gruppe | IL-2 | IFN-γ | IL-4 | IL-10 |
|---|---|---|---|---|
| A | 300 | 300 | 150 | 0 |
| B | 150 | 625 | 0 | 0 |
| C | 300 | 100 | 0 | 0 |
| D | 600 | 100 | 150 | 600 |

### BEISPIEL 12

### Behandlung von verstopfter Nase bei Freiwilligen mittels Influenzavirosomen mit und ohne HLT in einem neuen Sprayapplikator

In einer Klinik wurden 30 Freiwillige mit Erkältungssymptomen und akut verstopfter Nase ausgewählt. Bei sämtlichen Freiwilligen wurde die Atmungsintensität durch jedes einzelne Nasenloch rhinomanometrisch (in Pascal) gemessen. Anschließend wurden sie randomisiert und in 3 Gruppen zu 10 eingeteilt. Gruppe A erhielt eine Dosis Impfstoff (100 µl in jedes Nasenloch) gemäß Beispiel (1), Gruppe B erhielt die gleiche Dosis, jedoch ohne das mukosale Adjuvans HLT, Gruppe C erhielt je 100 µl 0,9% NaCl (physiologische Kochsalzlösung) in jedes Nasenloch. Die Luftströme wurden 15 min, 30 min, 1 Stunde sowie 2 Stunden später gemessen.

Die Messdaten sind in Tabelle 9 zusammengefaßt. Sowohl in Gruppe A und B waren die Werte signifikant besser als in Gruppe C. In beiden Gruppen konnte eine deutliche Besserung der Atemfunktionen festgestellt werden.

**Tabelle 9: Rhinomanometrische geometrische Mittelwerte bei Freiwilligen vor und nach Spray-Behandlung**

| Gruppe | Atmungsintensität in Pascal | | | | |
|---|---|---|---|---|---|
| | 0 min | 15 min | 30 min | 60 min | 120 min |
| A | 490 | 693 | 712 | 681 | 521 |
| B | 497 | 687 | 705 | 672 | 538 |
| C | 488 | 497 | 513 | 521 | 501 |

### BEISPIEL 13

### Behandlung von Mukosaläsionen in Freiwilligen mittels Infuenzavirosomen und HLT

Freiwillige wurden intranasal geimpft, wie im Bsp. (7) beschrieben. Wie hier beschrieben, wurden die durchschnittlichen Becherzellen 3, 7 und 28 Tage nach der intranasalen Vakzination bestimmt. Wir konnten Epithelveränderungen mit Becherzellhyperplasie in cytologischen Abstrichen bestimmen (Glück U, Gebber J-O: Nasal cytopathology in smokers: a possible biomarker of air pollution? Am. J. Rhinol. 10 (1996) 55-57). Becherzellen haben eine Schutzfunktion für die muköse Schicht. Einen Monat nach der ersten nasalen Vakzination war der zelluläre Zustand der Mukosa signifikant besser verglichen mit dem vorherigen Zustand.

Diese neue Vakzine kann daher als Therapeutikum zur Behandlung einer Nasenschleimhaut im geschädigten Zustand verwendet werden.

### BEISPIEL 14

### Prävention von enterotoxischer E. coli-Diarrhoe mittels nasaler Applikation von HLT mit und ohne Influenzavirosomen

Eine Gruppe von Reisenden, die Tunesien besuchten, bestand aus 38 Personen. Nach der informierten Einwilligung stimmten alle der Teilnahme an der Studie zu. Nach Randomisierung wurden 19 Freiwillige zweimal im Abstand von 1 Woche mit der Zubereitung gemäß Bsp. (1) geimpft, wogegen 19 Personen keinen Impfstoff erhielten. 28 Tage nach der ersten intranasalen Impfung wurden Blutproben von allen Freiwilligen abgenommen. Die 19 geimpften Personen zeigten hohe IgG-Spiegel im Serum gegen das mukosale Adjuvans (HLT). Die Kontrollgruppe blieb Anti-HLT-negativ. Bevor sie Tunesien verließen, einen Monat später, wurde ein spezielles Gesundheitsereignisformular an alle Teilnehmer verteilt. Die Gruppe kehrte 20 Tage später aus Tunesien zurück, und das ausgefüllte Gesundheitsereignisformular wurde auf Diarrhoeerkrankungen ausgewertet. In der Gruppe der Geimpften berichteten nur zwei Personen über Diarrhoeprobleme, wogegen in der nichtgeimpften Gruppe 9 Personen während des Aufenthalts in Tunesien an Diarrhoe litten. Diese Daten zeigen, daß die Vakzine auch zur Verhinderung von Diarrhoe der enterotoxischen *E*.-*coli*-Erkrankung wirksam ist.

**Beispiel 7: Tabelle 1: Klinisches Protokoll**

| Vakzin -Gruppe | N (männlich) | Mittleres Alter in Jahren | Anzahl der Vakzination en (Abstand) | Anwendung | Zusammensetzung in µg vollständ. Vakzination: HA pro Stamm | Zusammensetzung in µg vollständ. Vakzination: HLT |
|---|---|---|---|---|---|---|
| A | 20 (14) | 39,7 | 2 (1 Woche) | intranasal | 15 | 2 |
| B | 20 (14) | 35,5 | 2 (1 Woche) | intranasal | 15 | - |
| C | 20 (16) | 43,8 | 1 | intranasal | 15 | 2 |
| D | 20 (14) | 41,2 | 1 | intramuskulär | 15 | - |

## Patentansprüche

1. Manschette (24) mit einer Verbindungseinrichtung (28) zum Anordnen an einer Abgabeeinrichtung (2), insbesondere zur sprühenden Abgabe einer pharmazeutisch wirksamen Substanz, und einer Abstützeinrichtung (30), wobei die Verbindungseinrichtung (28) und die Abstützeinrichtung (30) derart angeordnet sind, daß sie zusammen einen optimalen Sprühwinkel (α) für die Abgabe der Substanz von der Abgabeeinrichtung definieren, wobei die Abstützeinrichtung (30) am benutzerseitigen Ende derart gebogen ist, daß sie im wesentlichen der Form eines Außenabschnitts der Oberlippe eines Benutzers entspricht, und wobei der Sprühwinkel (α) zwischen 50° und 80° zur Horizontalen liegt.

2. Manschette (24) nach Anspruch 1, wobei die Verbindungseinnchtung (28) ein im wesentlichen zylindrischer Rohrabschnitt ist.

3. Manschette (24) nach Anspruch 2, wobei der Rohrabschnitt (28) zumindest über einen Teil eines Nasenstücks (20) der Abgabeeinrichtung (2) schiebbar ist.

4. Manschette (24) nach einem der Ansprüche 1 bis 3, wobei die Manschette (24) drehfest an der Abgabeeinrichtung (2) angeordnet werden kann.

5. Manschette (24) nach Anspruch 4, wobei der Rohrabschnitt (28) und der Teil des Nasenstücks (20), über den der Rohrabschnitt (28) geschoben werden kann, zumindest teilweise polygonförmig ausgebildet sind, um eine Verdrehsicherung auszubilden.

6. Abgabeeinrichtung (2), insbesondere zur sprühenden Abgabe einer pharmazeutisch wirksamen Substanz, mit einer Pumpeinrichtung (6), einer Fingermanschette (10) zur Betätigung der Pumpeinrichtung (6), einem Anschlußelement (8) zum dichten Verbinden mit einem Vorratsbehälter (4) und einem Nasenstück (16), das einen ersten Abschnitt (18) und einen zweiten Abschnitt (20) aufweist, wobei die beiden Abschnitte (18, 20) des Nasenstücks (16) derart dimensioniert sind, daß eine Sprühöffnung (14) des Nasenstücks. (16) im wesentlichen bis zur Nasenhaupthöhle eines Patienten ragt, wobei die Abgabeeinreichtung (2) ferner eine Manschette (24) nach einem der Ansprüche 1 bis 5 aufweist.

7. Abgabeeinrichtung (2) nach Anspruch 6, wobei zumindest der zweite Abschnitt (20) des Nasenstücks (16) im wesentlichen zylindrisch ausgebildet ist.

8. Abgabeeinrichtung (2) nach Anspruch 7, wobei der Durchmesser des zweiten Abschnitts (20) zwischen 3 mm und 10 mm liegt, bevorzugt etwa 7 mm beträgt.

9. Abgabeeinrichtung (2) nach einem der Ansprüche 6 bis 8, wobei der zweite Abschnitt (20) des Nasenstücks (16) eine Länge zwischen 5 und 50 mm, bevorzugt etwa 20 mm aufweist.

10. Abgabeeinrichtung (2) nach einem der Ansprüche 6 bis 9, wobei der erste Abschnitt (18) des Nasenstücks (16) eine Länge zwischen 10 und 40 mm, bevorzugt etwa 30 mm aufweist.

11. Abgabeeinrichtung (2) nach einem der Ansprüche 6 bis 10, wobei die Distanz zwischen der Fingermanschette (10) und der an einer Spitze vorgesehenen Sprühöffnung (14) mindestens 30 mm, bevorzugt etwa 45 mm beträgt.

12. Abgabeeinrichtung (2) nach Anspruch 6, die einteilig beziehungsweise einstückig mit der Manschette (24) ausgebildet ist.

## Claims

1. A collar (24) with a connection element (28) for placing on an application device (2), in particular for spraying a pharmaceutically active substance, and a support means (30), wherein the connection element (28) and the support means (30) are placed in such a way that they form an optimum angle of spraying (α) for dispensing the substance with the application device, wherein the support means (30) at the user's end is curved in such a way that it essentially corresponds to the form of an outer section of the user's upper lip and wherein the angle of spraying (α) is between 50° and 80° to the horizontal line.

2. The collar (24) according to claim 1, wherein the connection element (28) is a substantially cylindrical tubular section.

3. The collar (24) according to claim 2, wherein the tubular section (28) may be put over at least a part of the nose piece (20) of the application device (2).

4. The collar (24) according to any one of claims 1 to 3, wherein the collar (24) is fixed to the application device (2) in such a way that it cannot rotate.

5. The collar (24) according to claim 4, wherein the tubular section (28) and the part of the nose piece (20) over which the tubular section (28) can be put have, at least in part, a polygon form in order to form a locking means.

6. An application device (2), in particular for spraying a pharmaceutically active substance, comprising a pump element (6), a finger collar (10) for activating the pump element (6), a connection element (8) for a tight connection with a storage container (4) and a nose piece (16) having a first section (18) and a second section (20), wherein the two sections (18, 20) of the nose piece (16) are formed in such a way that a spray outlet (14) of the nose piece (16) essentially extends to the main nasal cavities of a patient, said application device (2) further comprising a collar (24) according to any one of claims 1 to 5.

7. The application device (2) according to claim 6, wherein at least the second section (20) of the nose piece (16) essentially has a cylindrical form.

8. The application device (2) according to claim 7, wherein the diameter of the second section (20) ranges between 3 mm and 10 mm, preferably is approximately 7 mm.

9. The application device (2) according to any one of claims 6 to 8, wherein the second section (20) of the nose piece (16) has a length of 5 to 50 mm, preferably of approximately 20 mm.

10. The application device (2) according to any one of claims 6 to 9, wherein the first section (18) of the nose piece (16) has a length of 10 to 40 mm, preferably of approximately 30 mm.

11. The application device (2) according to any one of claims 6 to 10, wherein the distance between the finger collar (10) and the spray outlet (14) which is to be located at the top end is at least 30 mm, preferably approximately 45 mm.

12. The application device (2) according to claim 6 which is made in one piece or integrally connected with the collar (24).

## Revendications

1. Coupelle (24) comportant un dispositif de raccordement (28) à placer sur un dispositif de distribution (2), en particulier pour la distribution par pulvérisation d'une substance pharmaceutiquement active, et un dispositif d'appui (30), le dispositif de raccordement (28) et le dispositif d'appui (30) étant agencés de manière à définir ensemble un angle de pulvérisation (α) optimal pour la distribution de la substance par le dispositif de distribution, et le dispositif d'appui (30) étant, à son extrémité dirigée vers l'utilisateur, courbé de manière à correspondre substantiellement à la forme d'une section extérieure de la lèvre supérieure d'un utilisateur, l'angle de pulvérisation (α) étant de 50° à 80° par rapport à l'horizontale.

2. Coupelle (24) selon la revendication 1, où le dispositif de raccordement (28) est une section tubulaire substantiellement cylindrique.

3. Coupelle (24) selon la revendication 2, où la section tubulaire (28) peut coulisser sur au moins une partie d'un embout nasal (20) du dispositif de distribution (2).

4. Coupelle (24) selon l'une quelconque des revendications 1 à 3, où la coupelle (24) peut être placée sur le dispositif de distribution (2) de manière à ne pas subir de rotation.

5. Coupelle (24) selon la revendication 4, où la section tubulaire (28) et la partie de l'embout nasal (20) sur laquelle peut coulisser la section tubulaire (28) peut être au moins partiellement configurée en forme polygonale afin de former une sécurité empêchant une torsion.

6. Dispositif de distribution (2), en particulier pour la distribution par pulvérisation d'une substance pharmaceutiquement active, comportant un dispositif de pompage (6), une collerette (10) pour l'actionnement du dispositif de pompage (6) avec les doigts, un élément de raccordement (8) pour un raccord étanche à un récipient d'approvisionnement (4) et un embout nasal (16), présentant une première section (18) et une deuxième section (20), les deux sections (18, 20) de l'embout nasal (16) étant dimensionnées de manière qu'un orifice de pulvérisation (14) de l'embout nasal (16) atteigne substantiellement les fosses nasales d'un patient, le dispositif de distribution (2) présentant en outre une coupelle (24) selon l'une quelconque des revendications 1 à 5.

7. Dispositif de distribution (2) selon la revendication 6, où au moins la deuxième section (20) de l'embout nasal (16) est substantiellement cylindrique.

8. Dispositif de distribution (2) selon la revendication 7, où le diamètre de la deuxième section (20) est de 3 mm à 10 mm, de préférence d'environ 7 mm.

9. Dispositif de distribution (2) selon l'une quelconque des revendications 6 à 8, où la deuxième section (20) de l'embout nasal (16) présente une longueur de 5 à 50 mm, de préférence d'environ 20 mm.

10. Dispositif de distribution (2) selon l'une quelconque des revendications 6 à 9, où la première section (18) de l'embout nasal (16) présente une longueur de 10 à 40 mm, de préférence d'environ 30 mm.

11. Dispositif de distribution (2) selon l'une quelconque des revendications 6 à 10, où la distance entre la collerette (10) et l'orifice de pulvérisation (14) prévu à une extrémité est d'au moins 30 mm, de préférence d'environ 45 mm.

12. Dispositif de distribution (2) selon la revendication 6, formé en une partie ou en une pièce avec la coupelle (24).
